# EUROPEAN PATENT SPECIFICATION

(11) **EP 1 063 224 B1**
(45) Date of publication and mention of the grant of the patent: **25.06.2003**
(21) Application number: 00112634.1
(22) Date of filing: 15.06.2000
(51) Int. Cl.: C07C 67/31, C07C 69/675, C07C 69/68, C07D 307/32

(54) **Continuous hydrogenation process**
Kontinuierliches Hydrierverfahren
Procédé d'hydrogénation continue

(30) Priority: 23.06.1999 EP 99112078
(43) Date of publication of application: 27.12.2000
(73) Proprietor: F. HOFFMANN-LA ROCHE AG, 4070 Basel (CH)
(72) Inventor: Baiker, Alfons, 8152 Opfikon (CH); Kuenzle, Niklaus, 9500 Wil (CH); Mallat, Tamas, 8057 Zürich (CH)
(74) Representative: Kellenberger, Marcus Dr.

(56) References cited:
- US-A- 4 329 487
- PATENT ABSTRACTS OF JAPAN vol. 011, no. 398 (C-466) & JP 62 158268 A (NISSAN CHEM IND LTD), 14 July 1987 (1987-07-14)
- PATENT ABSTRACTS OF JAPAN vol. 004, no. 069 (C-011), 22 May 1980 (1980-05-22) & JP 55 035060 A (AGENCY OF IND SCIENCE & TECHNOL), 11 March 1980 (1980-03-11)

## Description

The present invention relates to a continuous process for the enantioselective hydrogenation of alpha ketocarbonyl compounds.

In particular the invention relates to a continuous process for the enantioselective hydrogenation of alpha-ketoesters and alpha-ketolactones.

US Patent No. 4329487 describes a method for the asymmetric hydrogenation of alpha-ketoesters which comprises subjecting an alpha-ketoester to asymmetric hydrogenation in the presence of a platinum-alumina catalyst modified with a solution of a cinchona-alkaloid selected from at least one member of the group consisting of quinine, quinidine, cinchonidine and cinchonine.

According to US 4329487 alpha-ketoesters are reacted by batch reaction accomplished in a pressure container such as an autoclave.

The hydrogenation of ketopantolactone over cinchonidine modified Pt-alumina catalyst in a batch process has been investigated and described by A. Baiker et al in Journal of Catalysis, 176, 569-571, (1998).

Japanese patent publication JP 55 035060 describes the asymmetric hydrogenation of alpha ketolactones in a batch process in the presence of a platinum-carbon catalyst modified by immersion in a solution of a (-)-quinine, (+)-quinidine, or cinchonine.

P.A.Meheux, A. Ibbotson, P.B.Wells, J. Catal., 128 (1991) 387, reported the enantioselective hydrogenation of ethyl pyruvate on Pt/Al₂O₃ in a continuously stirred tank reactor. They preadsorbed cinchonidine on the catalyst before use but did not feed the alkaloid during reaction.

The Japanese patent publication JP 62 158268 describes the asymmetric hydrogenation of alpha ketolactones in a batch process in the presence of a platinum-carbon catalyst modified with a solution of a cinchona-alkaloid selected from at least one member of the group consisting of quinine, cinchonine or cinchonidine. The preparation of the catalyst comprises e.g. mixing 0.5 g 5% Pt/C and 40 ml 1% cinchonidine/ethanol and refluxing said mixture for 3 h. The catalyst is separated with a centrifuge. A mixture of the catalyst and e.g. ketopantolactone in benzene is autoclaved to give D-pantolactone. The reaction temperature is 10 to 100°C, preferably room temperature. The hydrogen pressure is normal pressure to 100 kg/cm², preferably 60 kg/cm².

The drawback of the batch process is the huge reactor volume needed for reaction and solid-liquid separation. Another drawback of the batch process is the need of a stirrer which leads to mechanical abrasion of catalyst particles [M. Garland, H.P. Jalett and H.U. Blaser, *Stud. Surf. Sci. Catal*. 59 (1991) 177].

It is an object of the present invention to provide a process that overcomes the aforesaid drawbacks, and still preserves the high enantioselectivity characteristic of the batch process.

It has now been found that it is possible to carry out the hydrogenation of alpha-ketocarbonyl compounds continuously.

Thus, the present invention relates to a continuous process for catalytic hydrogenation of a substrate containing or consisting of an alpha ketocarbonyl compound which process comprises the steps of:
(i) contacting in a reactor, charged with a platinum catalyst, a substrate, a modifier for said platinum catalyst selected from a solution of a cinchona-alkaloid or derivatives thereof, 2-hydroxy-2-aryl-ethylamine or derivatives thereof, 1-aryl-ethylamine or derivatives thereof and hydrogen, optionally in the presence of a solvent and, further optionally, a supercritical co-solvent, at a temperature of from -20 °C to 100 °C and at pressures ranging from 2 bar to 150 bar to convert said alpha ketocarbonyl compound to the corresponding alpha hydroxy carbonyl compound;
(ii) continuously feeding said substrate and said modifier to the reactor;
(iii) continuously feeding hydrogen to the reactor;
(iv) continuously discharging the reaction product from the reactor; and
(v) recovering the alpha hydroxy carbonyl compound from the reaction product.

As used herein the term "substrate" refers to a solution of a solid alpha ketocarbonyl compound or to a liquid alpha ketocarbonyl compound. If appropriate the liquid alpha ketocarbonyl compound can be mixed with a solvent.

As used herein the term "alpha ketocarbonyl compound" refers to alpha ketolactones such as alpha ketopantolactone or to alpha-ketoesters such as e.g. esters of alpha (C₁-C₆) alkyl ketopropionic acid or esters of alpha aryl ketopropionic acid. An example of an alpha (C₁-C₆) alkyl ketopropionic acid ester is pyruvic acid ethyl ester (CH₃COCOOC₂H₅). An example of an alpha aryl ketopropionic acid ester is benzoylformic acid ethyl ester. (Phenyl-COCOOC₂H₅).

A preferred solid alpha ketocarbonyl compound is alpha ketopantolactone. A preferred liquid alpha ketocarbonyl compound is pyruvic acid ethyl ester.

Suitable solvents to dissolve the solid alpha ketocarbonyl compound include organic solvents and mixtures of organic solvents with water.

Suitable solvents to be mixed with a liquid alpha ketocarbonyl compound include organic solvents, supercritical solvents and mixtures of organic solvents with water.

Suitable organic solvents include aromatic solvents such as e.g. toluene, benzene, cumene; aliphatic solvents such as hexane, cyclohexane, pentane, cyclopentane, diethylether, tetrahydrofuran, acetic acid, alcohols, acetone, formamides and mixtures thereof. A preferred alcohol is e.g. ethanol or propanol. A preferred formamide is e.g. dimethylformamide.

Addition of small amounts (0.1-5 wt%) of carboxylic acid (e.g. acetic acid, trifluoracetic acid) or amine (e.g. triethyl amine, quinoline) can also be useful.

The choice of the solvent is not critical. Any solvent capable of dissolving the alpha ketocarbonyl compound can be used in this invention. If a solvent is present the reaction is preferably carried out in a supercritical state.

Suitable solvents or co-solvents for carrying out the reaction in a supercritical state are selected from the group consisting of methane, ethane, propane, carbon dioxide, sulfurhexafluoride, chlorinated- and fluorinated solvents and the like.

As used herein the term "platinum catalyst" refers to platinum metal deposited onto a variety of supports such as carbon black, calcium carbonate, activated alumina, silica or zeolithes. These catalysts are well known and commercially available. Suitable catalysts contain 0.5wt% to 10wt% of platinum. For example a catalyst containing 5wt% of platinum deposited onto alumina is sold by Engelhard Corp. with the code number 4759. The catalyst is charged into a fixed bed reactor. A metal loading of more than 5 wt% of platinum may require dilution of the catalyst bed with inert beads.

Suitable cinchona alkaloids are e.g., quinine, hydroquinine, cinchonidine, 10-11-dihydrocinchonidine, O-methyl-cinchonidine, 10-11 -dihydro-O-methyl-cinchonidine epiquinidine, epicinchonidine, cinchonine, epicinchonine, epiquinine, hydroquinidine, 4-chlorobenzoate-epiquinine or 4-chlorobenzoate-epicinchonine. Preferred is cinchonidine and dihydrocinchonidine.

Examples of 2-hydroxy-2-aryl-ethylamines or derivatives thereof are 2-(1-pyrrolidinyl)-1-(1-naphthyl)ethanol, 2-(1-pyrrolidinyl)-1-(4-azanaphthyl)ethanol and 2-(1-(N,N-dimethyl) amino)-1-(1-naphtyl)ethanol.

Examples of 1-aryl-ethylamines or derivatives thereof are 1-(1-naphtyl)-ethlyamine, 1-(1-naphtyl)-(N-methyl)-ethylamine, 1-(1-naphtyl)-(N-propyl)-ethylamine and N-[1'-(1-naphtyl)ethyl]-2-amino propionic acid ethyl ester.

All modifiers are amine bases. They can be used as free bases or as a salt with an acid, such as HCl, HClO₄, CF₃COOH, etc. A commercially available modifier is cinchonidine hydrochloride.

The modifier can be added to the substrate before starting the hydrogenation process, thus modifying the platinum catalyst when flowing through the reactor vessel or the platinum catalyst can be modified by immersing the platinum catalyst into a solution of the modifier before charging the reactor vessel with the catalyst. It is preferred to add the modifier to the substrate before starting the hydrogenation process.

The modifier is generally added in form of a solution. Any organic solvent capable of dissolving the modifier can be added e.g. organic solvents as defined above. Preferably the same solvent is used to dissolve the modifier and the alpha ketocarbonyl compound.

There is no limit of the reactor size as long as a sufficient heat transfer is guaranteed. A suitable reactor vessel consists of a 1 to 40 ml stainless steel or inconel tube heated with electrical heating tape or cooled with a cooling jacked. A thermocouple measures the temperature in the center of the tube. The catalyst bed consists, e.g., of 0.1 to 20 g of catalyst depending on the volume of the reactor. However, reactors of other types and size that are appropriate for conducting a continuous hydrogenation can be used.

At the end of the process the reaction product is discharged from the reactor vessel and the alpha hydroxy carbonyl compound recovered by methods well known in the art such as crystallization or distillation.

The invention will now be illustrated by the figures.

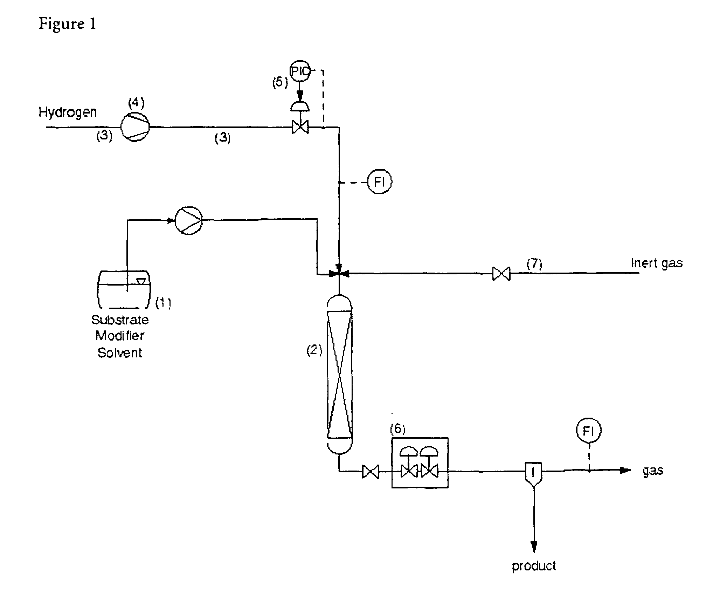

FIGURE 1 shows the flow diagram in schematic form of a hydrogenation apparatus according to one embodiment of the invention. A solid alpha ketocarbonyl compound is dissolved in an organic solvent, thus forming the substrate. The modifier is added to the substrate. Examples 1 and 2 refer to a hydrogenation process carried out according to FIGURE 1.

The process is typically initiated by dissolving the alpha ketocarbonyl compound and the modifier in vessel (1). The resulting solution contains from 0.1 wt% to 100wt% of alpha ketocarbonyl compound and from 1·10⁻⁵ wt% to 0.5 wt% of modifier.

The mass flow is started at reaction temperature, for example, as used in Examples 1 and 2, at 17 °C and 20 °C, respectively. The above solution containing alpha ketocarbonyl compound and modifier is pumped to the fixed bed reactor (2) and contacted with hydrogen to start the hydrogenation reaction. Before catalytic runs, the reactor is flushed with nitrogen.

Subsequently the content of vessel (1) is continuously pumped into the fixed bed reactor. The solution flow rate is preferably from 0.1 to 50 ml/min, the preferred flow of alpha ketocarbonyl compound is 2·10⁻⁵ - 2·10⁻² mol/g_{cat} /min. More preferably the solution flow rate is from 2.5 to 10 ml /min, and the flow of alpha ketocarbonyl compound from 2·10⁻⁴ to 3·10⁻³ mol/g_{cat} /min.

The modifier flow rate is preferably from 2·10⁻⁹ to 2·10⁻⁴ mol/g_{cat} /min, more preferably from 2·10⁻⁸ to 7·10⁻⁶ mol/g_{cat} /min.

Hydrogen is continuously fed into the fixed bed reactor via flow line (3) containing a compressor (4) and a pressure control system (5). The inert gas, e.g. nitrogen, is fed into the reactor (2) via line (7).

The hydrogen flow rate into the reactor is metered and monitored by a rotameter. Suitable hydrogen flow rates are from 0.0001 mol/min (2.4 ml/min) to 1 mol/min (24000 ml/min) and from 5·10⁻⁶ to 10 mol/g_{cat} /min.

The hydrogenation reaction can be carried out at a relatively low temperature ranging between -20 °C and 100 °C, the preferred range of temperature being from 0 °C to 20°C.

The pressure is suitably adjusted to between 2 bar and 150 bar, preferably from 40 bar to 100 bar.

The effluent from the hydrogenation reaction zone is fed over a two-step expansion module (6) to a separator where the alpha hydroxy carbonyl compound is recovered.

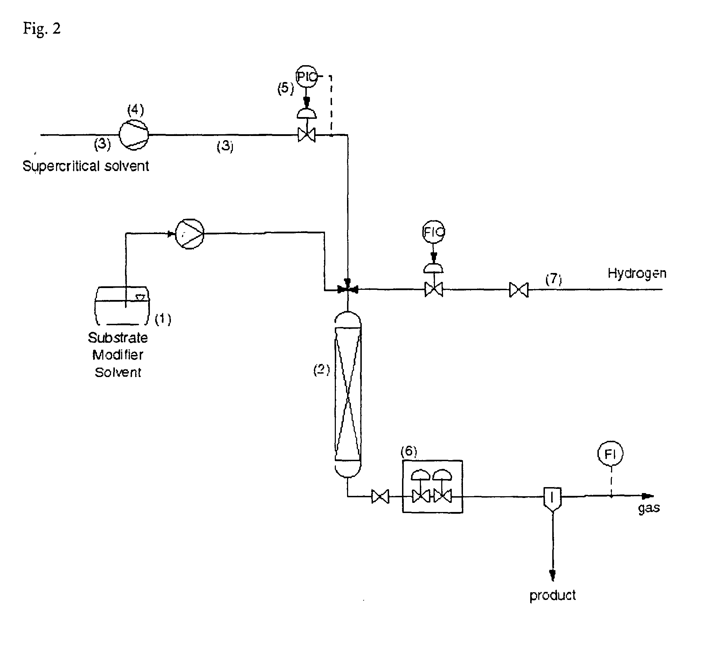

FIGURE 2 shows the flow diagram in schematic form of a hydrogenation apparatus according to another embodiment of the invention. The reactor vessel is charged with a supercritical solvent. Examples 3 and 4 refer to a hydrogenation carried out according to FIGURE 2.

The process is initiated by dissolving the alpha ketocarbonyl compound and the modifier in vessel (1) or by adding a solution containing the modifier to a liquid alpha ketocarbonyl compound. The resulting solution has the following concentration: 0.1 wt% to 100 wt% of alpha ketocarbonyl compound and 1·10⁻⁶ wt% to 0.5 wt% of modifier.

The reactor vessel (2) is charged with supercritical solvent via flow line (3) containing a compressor (4) and pressure control system (5).

The organic flow is started at reaction temperature for example as used in Examples 3 and 4 at 50 °C and 36 °C, respectively. The above solution is pumped to the fixed bed reactor (2) and contacted with hydrogen to start the hydrogenation reaction.

Subsequently the content of vessel (1) is continuously pumped into the fixed bed reactor with the same solution flow rate as in the process according to Fig.1.

The flow rate of the supercritical co-solvent is preferably from 50 ml/min to 5000 ml/min.

When using a liquid alpha ketocarbonyl compound the supercritical co-solvent is used with a flow rate of 50 ml/min to 5000 ml/min.

The modifier flow rate is preferably from 2·10⁻¹¹ to 2·10⁻⁴ mol/g_{cat} /min.

Hydrogen is continuously fed into the fixed bed reactor via flow line (7) containing pressure control system. The hydrogen flow rate into the reactor was metered and monitored by a rotameter.

Suitable hydrogen flow rates are from 0.0001 mol/min (2.4 ml/min) to 1 mol/min (24000 ml/min) and from 5·10⁻⁶ to 10 mol/g_{cat}/min.

The hydrogenation reaction can be carried out at a relatively low temperature ranging between 20 °C to 100 °C, the preferred range of temperature being 30°C to 60°C, and the most preferred range being 35°C to 50°C. The pressure is suitably adjusted to between 2 bar to 150 bar, preferably 40 bar to 100 bar.

To more fully demonstrate the advantages of the present invention, the following examples were performed. In the Examples, a fixed bed reactor of 38 ml volume was used.

| Example No. | 1 | 2 | 3 | 4 |
|---|---|---|---|---|
| Substrate | Alpha ketopantolactone | Pyruvic acid ethyl ester | Alpha ketopantolactone | Pyruvic acid ethyl ester |
| Solvent | Toluene | Toluene | Toluene/ Supercritical Ethane | Supercritical Ethane |
| Solvent flow (ml/min) | 5 | 5 | 5/4800 | 750 |
| Substrate flow (mol/min) | 3.9·10⁻⁴ | 7.6·10⁻⁴ | 3.9·10⁻⁴ | 4.3·10⁻³ |
| Modifier | Cinchonidine | | | |
| Modifier flow (mol/min) | 1.1·10⁻⁶ | 5.7·10⁻⁷ | 1.1·10⁻⁶ | 4.2·10⁻⁶ |
| Hydrogen flow (ml/min) | 80 | 80 | 1019 | 927 |
| Temperature (°C) | 17 | 20 | 50 | 36 |
| Pressure (bar) | 40 | 40 | 100 | 60 |
| Catalyst | 5 wt% Pt/alumina Engelhard 4759 | | | |
| Catalyst amount (g) | 1 | 1 | 1 | 0.5 |
| Conversion (%) | 100 | 86.4 | 100 | 95 |
| ee (%) | 79.7 | 89.9 | 62.1 | 74.8 |

## Claims

1. A continuous process for catalytic hydrogenation of a substrate containing or consisting of an alpha ketocarbonyl compound which process comprises the steps of:
(i) contacting in a reactor, charged with a platinum catalyst, a substrate, a modifier for said platinum catalyst selected from a solution of a cinchona-alkaloid or derivatives thereof, 2-hydroxy-2-aryl-ethylamine or derivatives thereof, 1-aryl-ethylamine or derivatives thereof and hydrogen, optionally in the presence of a solvent and, further optionally, a supercritical co-solvent, at a temperature of from -20 °C to 100 °C and at pressures ranging from 2 bar to 150 bar to convert said alpha ketocarbonyl compound to the corresponding alpha hydroxy carbonyl compound;
(ii) continuously feeding said substrate and said modifier to the reactor;
(iii) continuously feeding hydrogen to the reactor;
(iv) continuously discharging the reaction product from the reactor; and
(v) recovering the alpha hydroxy carbonyl compound from the reaction product.

2. A process according to claim 1, wherein the substrate is a liquid alpha ketocarbonyl compound or a solution of a solid alpha ketocarbonyl compound.

3. A process according to claim 2, wherein the liquid alpha ketocarbonyl compound is pyruvic acid ethyl ester and the solid alpha ketocarbonyl compound is alpha ketopantolactone.

4. A process according to any one of claims 1 to 3, wherein the solid alpha ketocarbonyl compound is dissolved in an organic solvent or mixture of organic solvents, or mixtures thereof with water.

5. A process according to any one of claims 1 to 3, wherein the liquid alpha ketocarbonyl compound is mixed with an organic solvent or mixtures of organic solvents, or mixtures thereof with water.

6. A process according to claims 4 and 5, wherein the organic solvent is toluene, benzene, cumene, hexane, cyclohexane, pentane, cyclopentane, diethylether, tetrahydrofuran, acetic acid, ethanol, propanol, acetone, dimethylformamide and mixtures thereof.

7. A process according to claims 1 and 5, wherein the solvent or co-solvent is methane, ethane, propane, carbon dioxide and sulfurhexafluoride.

8. A process according to any one of claims 1 to 7, wherein the platinum catalyst is platinum deposited onto carbon black, calcium carbonate, activated alumina, silica or zeolithes.

9. A process according to claim 8, wherein the platinum catalyst contains 0.5wt% to 10 wt% of platinum deposited onto alumina and the modifier is a solution of cinchonidine or dihydrocinchonidine.

10. A process according to any one of claims 1 to 9, wherein the substrate solution contains from 0.1 wt% to 100 wt% of alpha ketocarbonyl compound and from 1·10⁻⁶ wt% to 0.5 wt% of modifier.

11. A process according to any one of claims 1 to 10, wherein the hydrogenation reaction is carried out without a supercritical co-solvent at a temperature of from 0 °C to 20°C and at a pressure of from 40 bar to 100 bar.

## Patentansprüche

1. Kontinuierliches Verfahren zur katalytischen Hydrierung eines Substrats, das eine α-Ketocarbonylverbindung enthält oder aus dieser besteht, wobei das Verfahren die Stufen umfasst, dass
(i) in einem Reaktor, der mit einem Platinkatalysator beschickt ist, ein Substrat, ein Modifikator für den Platinkatalysator ausgewählt aus einer Lösung eines Cinchonaalkaloids oder von Derivaten davon, 2-Hydroxy-2-arylethylamin oder Derivaten davon, 1-Arylethylamin oder Derivaten davon und Wasserstoff, gegebenenfalls in Gegenwart eines Lösungsmittels und weiterhin gegebenenfalls eines superkritischen Co-Lösungsmittels bei einer Temperatur von -20 bis 100°C und bei einem Druck im Bereich von 2 bar bis 150 bar in Kontakt gebracht wird, um die α-Ketocarbonylverbindung in die entsprechende α-Hydroxycarbonylverbindung umzuwandeln;
(ii) kontinuierlich das Substrat und der Modifikator dem Reaktor zugeführt wird;
(iii) kontinuierlich Wasserstoff dem Reaktor zugeführt wird;
(iv) kontinuierlich das Reaktionsprodukt aus dem Reaktor entnommen wird und
(v) die α-Hydroxycarbonylverbindung aus dem Reaktionsprodukt gewonnen wird.

2. Verfahren nach Anspruch 1, wobei das Substrat eine flüssige α-Ketocarbonylverbindung oder eine Lösung einer festen α-Ketocarbonylverbindung ist.

3. Verfahren nach Anspruch 2, wobei die flüssige α-Ketocarbonylverbindung Brenztraubensäureethylester ist und die feste α-Ketocarbonylverbindung α-Ketopantolacton ist.

4. Verfahren nach einem der Ansprüche 1 bis 3, wobei die feste α-Ketocarbonylverbindung in einem organischen Lösungsmittel oder einer Mischung von organischen Lösungsmitteln oder Mischungen davon mit Wasser gelöst wird.

5. Verfahren nach einem der Ansprüche 1 bis 3, wobei die flüssige α-Ketocarbonylverbindung mit einem organischen Lösungsmittel oder Mischungen von organischen Lösungsmitteln oder Mischungen davon mit Wasser vermischt wird.

6. Verfahren nach Anspruch 4 und Anspruch 5, wobei das organische Lösungsmittel Toluol, Benzol, Cumol, Hexan, Cyclohexan, Pentan, Cyclopentan, Diethylether, Tetrahydrofuran, Essigsäure, Ethanol, Propanol, Aceton, Dimethylformamid und Mischungen davon ist.

7. Verfahren nach Anspruch 1 und Anspruch 5, wobei das Lösungsmittel oder Co-Lösungsmittel Methan, Ethan, Propan, Kohlendioxid und Schwefelhexafluorid ist.

8. Verfahren nach einem der Ansprüche 1 bis 7, wobei der Platinkatalysator Platin abgeschieden auf Ruß, Calciumcarbonat, aktiviertem Aluminiumoxid, Siliciumdioxid oder Zeolithen ist.

9. Verfahren nach Anspruch 8, wobei der Platinkatalysator 0,5 bis 10 Gew.-% Platin abgeschieden auf Aluminiumoxid enthält und der Modifikator eine Lösung von Cinchonidin oder Dihydrocinchonidin ist.

10. Verfahren nach einem der Ansprüche 1 bis 9, wobei die Substratlösung 0,1 bis 100 Gew.-% α-Ketocarbonylverbindung und 1 x 10⁻⁶ bis 0,5 Gew.-% Modifikator enthält.

11. Verfahren nach einem der Ansprüche 1 bis 10, wobei die Hydrierungsreaktion ohne ein superkritisches Co-Lösungsmittel bei einer Temperatur von 0 bis 20°C und bei einem Druck von 40 bis 100 bar durchgeführt wird.

## Revendications

1. Procédé continu pour l'hydrogénation catalytique d'un substrat contenant ou consistant en un composé α-cétocarbonyle, comprenant les étapes suivantes :
(I) mise en contact dans un réacteur, contenant un catalyseur à base de platine, d'un substrat, d'un agent modificateur pour ledit catalyseur à base de platine choisi parmi une solution d'un cinchona-alcaloïde ou de dérivés de celui-ci, d'une 2-hydroxy-2-aryl-éthylamine ou de dérivés de celle-ci, d'une 1-aryl-éthylamine ou de dérivés de celle-ci et d'hydrogène, éventuellement en présence d'un solvant et, encore éventuellement, d'un co-solvant supercritique, à une température de -20°C à 100°C et sous des pressions allant de 2 bars à 150 bars, pour convertir ledit composé α-cétocarbonyle en le composé α-hydroxycarbonyle correspondant ;
(II) introduction continue dudit substrat et dudit agent modificateur dans le réacteur ;
(III) introduction continue d'hydrogène dans le réacteur ;
(IV) décharge continue du produit de réaction du réacteur ; et
(V) récupération du composé α-hydroxycarbonyle à partir du produit de réaction.

2. Procédé selon la revendication 1, dans lequel le substrat est un composé α-cétocarbonyle liquide ou une solution d'un composé α-cétocarbonyle solide.

3. Procédé selon la revendication 2, dans lequel le composé α-cétocarbonyle liquide est le pyruvate d'éthyle et le composé α-cétocarbonyle solide est l'α-cétopantolactone.

4. Procédé selon l'une quelconque des revendications 1 à 3, dans lequel le composé α-cétocarbonyle solide est dissous dans un solvant organique ou un mélange de solvants organiques, ou des mélanges de tels solvants et d'eau.

5. Procédé selon l'une quelconque des revendications 1 à 3, dans lequel le composé α-cétocarbonyle liquide est mélangé avec un solvant organique ou des mélanges de solvants organiques, ou des mélanges de tels solvants et d'eau.

6. Procédé selon les revendications 4 et 5, dans lequel le solvant organique est le toluène, le benzène, le cumène, l'hexane, le cyclohexane, le pentane, le cyclopentane, l'oxyde d'éthyle, le tétrahydrofuranne, l'acide acétique, l'éthanol, le propanol, l'acétone, le diméthylformamide et des mélanges de ceux-ci.

7. Procédé selon les revendications 1 et 5, dans lequel le solvant ou co-solvant est le méthane, l'éthane, le propane, le dioxyde de carbone et l'hexafluorure de soufre.

8. Procédé selon l'une quelconque des revendications 1 à 7, dans lequel le catalyseur à base de platine consiste en du platine déposé sur du noir de carbone, du carbonate de calcium, de l'alumine activée, de la silice ou des zéolithes.

9. Procédé selon la revendication 8, dans lequel le catalyseur contenant du platine contient 0,5 % en poids à 10 % en poids de platine déposé sur de l'alumine et l'agent modificateur est une solution de cinchonidine ou dihydrocinchonidine.

10. Procédé selon l'une quelconque des revendications 1 à 9, dans lequel la solution de substrat contient de 0,1 % en poids à 100 % en poids de composé α-cétocarbonyle et de 1.10⁻⁶ % en poids à 0,5 % en poids d'agent modificateur.

11. Procédé selon l'une quelconque des revendications 1 à 10, dans lequel la réaction d'hydrogénation est effectuée sans un co-solvant supercritique à une température de 0°C à 20°C et sous une pression de 40 bars à 100 bars.
